# EUROPÄISCHE PATENTSCHRIFT

(11) **EP 2 051 685 B1**
(45) Veröffentlichungstag und Bekanntmachung des Hinweises auf die Patenterteilung: **13.04.2011**
(21) Anmeldenummer: 07788312.2
(22) Anmeldetag: 08.08.2007
(51) Int. Cl.: A61K 6/083

(54) **PASTENFÖRMIGE, POLYMERISIERBARE DENTALMASSEN UND VERFAHREN ZU DEREN HERSTELLUNG**
PASTY, POLYMERIZABLE DENTAL COMPOUNDS AND METHOD FOR PRODUCING THE SAME
MATÉRIAUX DENTAIRES POLYMÉRISABLES SE PRÉSENTANT SOUS FORME DE PÂTE ET PROCÉDÉ DE FABRICATION DE CES MATÉRIAUX

(30) Priorität: 16.08.2006 DE 102006038280; 30.07.2007 DE 102007035735
(43) Veröffentlichungstag der Anmeldung: 29.04.2009
(73) Patentinhaber: Ivoclar Vivadent AG, 9494 Schaan (LI)
(72) Erfinder: KAMMANN, Axel, A-6800 Feldkirch (AT); RHEINBERGER, Volker, CH-9490 Vaduz (LI)
(74) Vertreter: Fitzner, Uwe
(86) Internationale Anmeldenummer: PCT/EP2007/058230
(87) Internationale Veröffentlichungsnummer: WO 2008/019978

(56) Entgegenhaltungen:
- EP-A- 0 212 193
- EP-A- 0 584 376
- FR-A- 2 648 345
- JP-A- 58 072 509
- JP-A- 61 050 906

## Beschreibung

Die vorliegende Erfindung betrifft Ein- und Mehrpastensysteme für Dentalprodukte, ein Verfahren zu deren Herstellung sowie deren Verwendung.

Die gebräuchlichste und am meisten verbreitete Herstellung von Prothesen beschränkt sich auf die Verwendung von Polymer/Monomersystemen (Pulver/ Flüssigkeitssysteme), welche in getrennten Behältern aufbewahrt werden. Vor der Anwendung werden beide Komponenten, das Polymer und das Monomer, in einem bestimmten Mischungsverhältnis gemischt. Nach der Mischung entsteht, bedingt durch die Löslichkeit des Polymeren im Monomer, eine teigartige, knetbare Masse, mit stark eingeschränkter Lagerstabilität, welche nach bekannter Stopf-, Gieß- oder Injektionstechnik in eine Küvette gestopft, gegossen oder injiziert und anschließend in dieser ausgehärtet wird.

Dieses einfache Mischverfahren von MMA/PMMA-Systemen hat sich über Jahrzehnte hinweg bewährt. Die Gründe für die große Akzeptanz liegen nicht nur in den wirtschaftlichen Vorteilen, sondern auch in der klinischen Beständigkeit und den mechanischen Eigenschaften, welche form- und härtbare Polymer/ Monomersysteme auf dieser Basis aufweisen.

Monomer/Polymermischungen gemäß dem Pulver/Flüssigkeitssystem besitzen aber sowohl als Mischung, als auch ausgehärtet Nachteile. Bei dem Pulver/ Flüssigkeitssystem werden Polymer- und Monomermischung individuell zusammengerührt, d.h. der Zahntechniker beeinflusst durch seine persönliche Mischtechnik die Eigenschaft und Güte der Mischung und damit der fertig gestellten polymerisierten Prothese. Das System beinhaltet eigentlich zwei Mischvorgänge: Zum ersten erfolgt das Einrühren der Pulverkomponente (Polymer) in das Monomer und zweitens nach dem Reifeprozess durch individuelles Durchkneten der Mischung von Hand. Nach dem Einrühren der Pulverkomponente in die Flüssigkeit kann es zu Sedimentationsvorgängen und Inhomogenitäten kommen. Nach dem Einrühren der Pulverkomponente beginnen gleichzeitig an den Grenzflächen der Polymerpartikel Quell- und Lösungsvorgänge, welche zu einer Viskositätserhöhung der gesamten Mischung führen. Ein Teil der polymeren Bestandteile lösen sich in der Monomerphase und verwandeln diese zuerst über eine sirup- oder honigartige Konsistenz in einen, plastisch verformbaren Teig.

Bricht der Zahntechniker diesen Reifeprozess zu früh ab, entsteht eine so genannte Magermischung, in welcher die Partikel zu wenig von der Flüssigkeit gebunden werden. Beim Auspressen der mager angeteigten Masse werden die Partikel in der Küvette von der flüssigen Phase getrennt, dabei kommt es zu Farbproblemen und ungenügenden, mechanischen Eigenschaften. Die Bildung dieser teigartigen Paste oder Masse muss in einem geschlossenen Gefäß stattfinden, ansonsten verursachen die ausgetrockneten Partikel des Teiges, besonders bei autopolymerisierenden Systemen, häufig Blasennester, mürbe Randpartien und fehlerhaften Zahnverbund.

Wird der Quellvorgang im geschlossenen Gefäß zu lange abgewartet, wird der Teig überreif: Die plastische Verformbarkeit nimmt ab, verhindert das funktionsgerechte Auspressen der Masse und führt zur Faltenbildung und ebenfalls zu mangelhaftem Zahnverbund.

Ist die teigartige Masse einmal in der Küvette verpresst, wird der kontinuierlich fortschreitende Quell- und Lösevorgang der Polymer-/Monomermischung in der Küvette durch eine Erwärmung beschleunigt. Kurz vor Beginn der radikalischen Polymerisation entsteht eine lederzähe, hornartige Masse, die durch die Erwärmung und die dadurch bedingte thermische Expansion eine Zunahme des Druckes in der Küvette ihre Form nicht mehr verliert.

Während der Polymerisation wandelt sich die ehemals hochviskose Polymerlösung in eine stabile und feste Polymermatrix, welche die Partikel des Polymeren nun fest umschließen bzw. im Gefüge festhalten. Diese Polymerpartikel enthalten häufig Defekte, wie Luftblasen und vernetzte Anteile. Beim Fräsen und Beschleifen der Prothesenoberflächen werden diese Defekte freigelegt. Es entstehen mikroskopisch kleine Poren, in denen sich beim Tragen bevorzugt ein Biofilm bildet, auf dem sich in der Folge Bakterien ablagern.

Im Hinblick auf die beschriebenen Schwierigkeiten in der Handhabung von Pulver-/Flüssigkeits-Systemen sind Ein- und Mehrkomponentensysteme entwickelt worden.

Unter Einkomponentenmaterialien versteht man reaktionsfähige Massen, welche die monomeren Bestandteile, die feste Pulverkomponente und die Initiatoren, welche zur initiierung der Polymerisation dieser reaktionsfähigen Monomere nötig sind, alle schon enthalten. Im Gegensatz zu den Pulver/-Flüssigkeitssystem ist die Masse gebrauchsfertig, ohne dass durch weiteres Zumischen eine oder mehrere Komponente hinzugefügt werden müssen. Die Schwierigkeit besteht darin, die Monomere vor frühzeitiger Polymerisation so zu stabilisieren, dass diese über längere Zeit (ca. 6 Monate, bei 28°C) lagerfähig bleibt.

Zu den gebräuchlichen Einkomponentensystemen gehören heißpolymerisierbare Materialien auf Basis Vinylchlorid/vinylacetat (VC/VAc)-Mischpolymerisat, die unter dem Namen LUXENE^{®} und LUXIDENT^{®} bekannt sind. Als Katalysator wird Benzoylperoxid eingesetzt. Die Nachteile der betreffenden Materialien liegen im hohen Preis, im komplizierten Verfahren, in der schlechten Lagerstabilität sowie der Verfärbungsneigung während bzw. nach der Polymerisation.

Die aus dem Stand der Technik bekannten Methylmethacrylat (MMA) enthaltenden Pulver/Flüssigkeitssysteme weisen Nachteile auf. Denn MMA ist flüchtig und hinterlässt ohne spezielle Verpackungsmaßnahmen, nach längerer Handhabung an Luft oder in undichten Gefäßen eine ausgetrocknete Masse, welche sich schlecht verarbeiten lässt und auf Grund des unkontrollierten Verlustes an MMA nach der Polymerisation unterschiedliche Eigenschaften aufweist.

Aus diesem Grund wurden Einkomponentenmaterialien ohne Methylmethacrylat entwickelt. Solche Materialien sind beispielsweise in der US 5,502,087, EP 0 630 641 A1 und EP 0 687 451 A2 beschrieben. Z.B. werden Einkomponentenmaterialien auf Basis von di- oder mehrfachfunktionellen (Meth)acrylaten hergestellt, welche vernetzte Polymerisate auf Polymethylmethacrylat (PMMA)- Basis und/oder anorganische Füllstoffe enthalten. Phlegmatische Initiatoren wie längerkettige Perester oder in vernetzte PMMA- Perlen einpolymerisierte Initiatoren schützen vor frühzeitiger Polymerisation. Derartige Systeme sind deswegen nicht lagerstabil, weil das enthaltene Perlpolymerisat im Laufe der Lagerung anteigt und so zur Verstrammung führt. Abgesehen davon weisen die bekannten heißhärtenden Materialien noch weitere Nachteile auf: Sie sind sehr spröde, schlecht polierbar und klinisch nicht beständig.

Ferner sind aber auch Materialien auf Basis von MMA/PMMA (INJECTALL^{®}, Fricke) bekannt. Derartige heißhärtende Einkomponentenmaterialien sind aus einem schlagzähmodifizierten Suspensionspolymerisat zusammengesetzt, welches in MMA gequollen wurde. Der Teig erreicht nach dem Lösen des Polymerisates eine Viskosität, welche während der Lagerung über einen längeren Zeitraum hinweg konstant bleibt. Eine derartige Mischung ist aber für die universelle Stopftechnik nicht geeignet, da für eine ausreichende Verarbeitbarkeit der Gehalt an Polymer nur 40-50% betragen darf. Das Material wird auf Grund der beschränkten Lagerstabilität auf Bestellung zubereitet und ausgeliefert.

Aus dem Stand der Technik sind auch lichthärtende Einkomponenten-materialien bekannt. Solche lichthärtenden Kompositmaterialien enthalten kein MMA und sind wie ihre heißhärtenden Pendenten ebenfalls vor Austrocknung geschützt. Die Nachteile sind hohe Sprödigkeit, die schlechte Polierbarkeit, die Inhibierung der basalen Oberfläche und die schlechte klinische Stabilität.

Lichthärtende Materialien sind kritisch, weil die Lichtquelle der bekannten Systeme nicht alle Stellen gleichmäßig erreicht und damit aushärtet. Außerdem ist die Haftung der Zähne an solchen Materialien ungenügend.

Weiterhin sind selbsthärtende Pastensysteme bekannt (z.B. IVOPAST® IVAG). Diese Pastensysteme bestehen aus mindestens zwei Pasten, in denen die Initiatoren (z.B. Peroxide) und die Beschleuniger (z.B. Amine) getrennt enthalten sind. Derartige Pastensysteme sind als Füllungsmaterialien, Kronen- und Brückenmaterialien auf Kompositbasis und dualhärtende Aufbaukomposite und Zemente wohl bekannt. ZweipastenSysteme für die Herstellung von Prothesen sind dagegen nicht bekannt.

Gemäß dem oben erwähnten Stand der Technik sind solche Pasten, welche als zahntechnische Massen verarbeitet werden, so zu stabilisieren und phlegmatisieren, dass eine Lagerstabilität für mindestens 1 Jahr bei einer Durchschnittstemperatur von 23 ± 5 °C gewährleistet ist. Durch die Phlegmatisierung, sei es durch Zugabe von phlegmatisierenden Substanzen wie Stabilisatoren, oder durch Einsatz von phlegmatisierten Peroxiden, welche erst bei relativ hoher Temperatur Radikale bilden, besteht die Gefahr einer zu geringen Polymerisation und einer weißlichen Fleckenbildung durch hohen Restmonomergehalt. Damit die Qualität dieser Prothesenmaterialien akzeptabel wird, müssen längere Prozesszeiten in Kauf genommen werden, um das Material genügend auszuhärten, oder die Lagerstabilität wird durch eine konsequente Kühlung des Produktes bis zum Verbrauch gewährleistet.

Ein anderes Problem ist das Handling solcher Pastensysteme. Um die Viskosität über den erwähnten Zeitraum hinaus konstant zu halten müssen die (Co)-Polymerisate aus PMMA vollständig gelöst, oder unlösliche Feststoffpartikel in einem relativ hohen Gehalt an Monomer dispergiert werden. Dadurch schrumpft solch ein System mehr, als dies bei bekannten Pulver/Flüssigkeitsgemischen aus Zweikomponenten- Prothesenmaterialien der Fall ist. Ein solches Pastensystem ist dann von einem speziellen Heißhärtungs-Applikationsverfahren, wie z.B. einem Injektionssystem oder der Lichthärtung abhängig.

Aus der DE-OS, 36 08 409 ist es bekannt, technische Polysulfone in thermoplastischen Prothesenmaterialien zu verwenden. Diese Thermoplasten werden analog dem Spritzguss verarbeitet, wodurch hohe Kosten für die notwendige Technik entstehen. Im Gegensatz zu den bekannten Pulver/Flüssigkeitssystemen weisen diese Materialien gravierende Nachteile auf: Der unvernetzte Thermoplast ist beim Tragen der Prothese im Mund anfällig auf Spannungsrisskorrosion und besitzt eine ungenügende Haftung zu den eingebetteten Zähnen. Der Kunststoff lässt sich außerdem schlecht vom Gips trennen, da unter hohem Einspritzdruck und bei der hohen Verarbeitungstemperatur der Gips örtlich zersetzt wird und die abgebundenen Alginattrennmittel nicht mehr wirksam sind.

Aus der EP 0760249 B1 ist ein Verfahren zur Herstellung von verbundenen Mikrogelteilchen und mit verbundenen Mikrogelteilchen behandelten Gegenständen bekannt. Es entsteht ein homogener Verbund durch die Ausbildung von interpenetrierenden Polymernetzwerken. Die Herstellung von Dentalprothesen wird in dieser Schrift nicht offenbart.

Aus der WO 2004/113042 A2 ist eine Zusammensetzung bekannt, die für das Rapid Prototyping verwendet werden können. Die Materialien zeichnen sich dadurch aus, dass feste Polymerpartikel oberflächlich mit einem Monomer mit adhäsiven Eigenschaften benetzt und anschließend polymerisiert werden. Vor der Penetration werden die Monomere gelöst. Beispielsweise können Polysulfone oder Polyethersulfone in Diglyciclylether von Bisphenol A gelöst werden.

In der US 2,793,436 wird die Herstellung von künstlichen Zähnen oder Zahnteilen beschrieben. Hierbei werden anorganische Fasern als Verstärkungsmittel in eine Monomermischung eingearbeitet und zu einer Paste weiterverarbeitet.

Aus der EP 059525 B1 sind Dentalmischungen bekannt, in denen neben löslichen Polymerern noch große Anteile eines unlöslichen (quellbaren) vernetzten Polymers verwendet werden. Das voll gequollene vernetzte Polymer wird in einem zweiten Schritt zu einer fertigen Paste unter Zusatz von Acrylsäure und Kielgel gemischt. Der Einsatz von Polysulfonen ist aus diesem Stand der Technik nicht bekannt.

Polysulfone werden schon seit Jahrzehnten in der Medizintechnik, der optischen Industrie und der Elektrotechnik eingesetzt. Aus der US 6,605,651 B1 ist beispielsweise der Einsatz von Polysulfonen als thermoplastischer Polymerbestandteil in polymerisierbaren Dentalmatten vorgeschlagen worden. Allerdings wird Polysulfon als eines von mehreren einsetzbaren thermoplastischen Komponenten erwähnt. Nähere Ausführungen zur Verarbeitung von Polysulfonen sind nicht vorhanden. Gerade für den Einsatz in der Medizintechnik wurden FDA- Zulassungen für eine große Bandbreite von Polysulfon- Typen erteilt.

Die vorliegende Erfindung hat sich nunmehr die Aufgabe gestellt, insbesondere die aus dem Stand der Technik bekannten Nachteile von Einkomponentensystemen zu verbessern. Die erhaltenen Materialien sollen sich insbesondere durch verbesserte Handhabungseigenschaften auszeichnen. Außerdem sollen Biegefestigkeit, Bruchfestigkeit, Transparenz sowie die chemische Beständigkeit optimiert werden. Die Zielrichtung der vorliegenden Erfindung ist jedoch nicht auf Einkomponentensysteme beschränkt. Vielmehr sollen auch Systeme zur Verfügung gestellt werden, bei denen z. B. die Initiatoren gesondert gelagert und im Falle der Verarbeitung zugeführt werden.

Gelöst wird diese Aufgabe durch Verformbare Massen enthaltend eine Mischung aus
- 20 bis 50 Gew.-% wenigstens eines polymerisationsfähigen, für Dentalzwecke geeigneten Monomers und
- 50 bis 80 Gew.-% wenigstens eines für Dentalzwecke geeigneten Polymers auf Basis von Polysulfon, das wenigstens teilweise in wenigstens einem Teil des Monomers gequollen ist,
   wobei sich die Anteile auf jeweils 100 Gew.-% addieren und wobei die Masse als polymerisationsfähige Monomere Mono(meth) acrylate oder kurzkettige Dimethylacrylate oder Gemische hiervon enthält. Gegenstand der Erfindung ist ferner ein Verfahren zur Herstellung von verformbaren Massen, wobei
- 20 bis 50 Gew.-% wenigstens eine polymerisationsfähigen, für Dentalzwecke geeigneten Monomers,
- 50 bis 80 Gew.-% wenigstens eines für Dentalzwecke geeigneten Polymers auf Basis von Polysulfon vermischt werden, und
- wenigstens teilweise das Polymer in wenigstens einem Teil des Monomers gequollen wird.
wobei sich die Anteile auf jeweils 100 Gew.-% addieren.

Ebenfalls ist Gegenstand der vorliegenden Erfindung die Verwendung der erfindungsgemäßen verformbaren Massen sowie der nach dem erfindungsgemäßen Verfahren hergestellten Produkte zur Herstellung von Dentalprodukten, vorzugsweise zur Herstellung von Dentalprothesen.

Die verformbaren Massen liegen insbesondere in Form von fließfähigen Systemen vor. Vorzugsweise kann es sich um Flüssigkeiten, Dispersionen, Suspensionen, Schmelzen, Feststoffgemische, thermoplastische Systeme handeln. Besonders bevorzugt ist der Einsatz der erfindungsgemäßen verformbaren Massen in Form von Pasten.

Bevorzugte Zusammensetzungen der erfindungsgemäßen verformbaren Massen enthalten zwischen 55 und 75 Gew.-% Polymer und zwischen 25 und 45 Gew.-% Monomer.

Zu den bevorzugten reaktionsfähigen Monomeren gehören Methylmethacrylat, Ethylmethacrylat, Butylmethacrylat, Ethylacrylat, Butylacrylat, Hydroxyethyl(meth-)acrylatmonomethylether, Neopentyl-glycolpropoxyloxy-methylethermono(meth-)acrylat, Ethylglycoldimeth-acrylat, Butandioldimethacrylat, ethoxyliertes Bisphenol-A-Dimethacrylat (SR 348C), ethoxyliertes Cyclohexandimethanoldimethacrylat.

Im Gegensatz zu Polycarbonat, Polyether(ether-)-keton, Polyetherimid, Polyether (ether)-sulfon sind die Polysulfone in Mono(meth-)acrylaten und kurzkettigen, polaren Di(meth-)acrylaten gut quellbar oder sogar löslich. Durch einen bei 60°C in der Wärme beschleunigten Quell- oder Lösungsvorgang, lässt sich das Polysulfon-Granulat oder Pulver in den Monomeren quellen und auflösen.

Die Verteilung der Komponenten erfolgt in geeigneten Mischern, wie z.B. einem Z-Arm-Kneter. Die dabei auftretenden Scherkräfte und die durch die Bewegungsenergie entstehende Reibungswärme verursacht eine gleichmäßige Verteilung der Polymerphase im Monomer.

Technisch gebräuchliche Polysulfone mit niedrigen Molmassen von ca. 20 kDa bis ca. 32 kDa sind als Polymerkomponente in den o.g. Monomeren zur Herstellung von Prothesenmaterialien geeignet und sind in den mechanischen Eigenschaften den im Pulver/-Flüssigkeitsverfahren hergestellten MMA/PMMA- Materialien gleichwertig oder sogar überlegen. Die Biegefestigkeit und die Bruchzähigkeit sind hierbei als besondere Merkmale herauszuheben, aber auch die Transparenz und die Beständigkeit gegenüber heißen, wässerigen Farbstofflösungen und Nahrungsmittelbestandteilen ist denjenigen von PMMA- (Meth-)acrylat Kompositionen wenigstens gleichwertig. Die geringen Molmassen der Polysulfone und die daraus hergestellten polymerisierbaren Massen garantieren selbst bei einem Polymergehalt von bis 75 Gew.-% für den Zahntechniker gute Handlingseigenschaften.

Die beschriebenen, polymerisierbaren Massen können für verschiedene Systeme eingesetzt werden.

Mögliche Ausführungsformen sind Ein- und Zweipastensysteme. Neben einem Einpastenmaterial, welches nur aus einer Komponente besteht und mit Hitze ausgehärtet werden kann, sind auch Zweipastensysteme möglich.

Die Einpastensysteme enthalten nur einen durch Energiezufuhr aktivierbaren Initiator. Als Initiatoren kommen vorzugsweise Peroxide, wie Dibenzoylperoxid, Dilauroylperoxid, tert.-Butylperoctoat, tert.-Butylperbenzoat oder Di-(tert.-butyl)-peroxid in Betracht. Typische Zusammensetzungen enthalten phlegmatisierte Peroxide, Peroxyester, Perketale oder Nitrile (Dibenzoylperoxid, Dilauroylperoxid, Di-tert-butylperoxy- (trimethyl-)cyclohexan, Tert-butyl-peroxyethylhexanoat, 1,1'- azobis (cyclohexan-carbonitril)).

Im Gegensatz dazu enthalten die Zweipastensysteme die Komponenten des Initiatorsystems aufgeteilt auf die beiden Pasten: Die eine Paste ist mit dem zur Spaltung nötigen Redox-Beschleuniger (Aktivator) versehen (z.B. tertiäre Amine wie N,N-Dimethyl-p-toluidin, N,N-Dihydroxyethyl-p-toluidin, p-Dimethylamino-benzoesäure-ethylester oder strukturverwandte Systeme wie Xylidine oder Aniline geeignet (N,N'- Dimethylsym-xylidin, N,N'- Di (2- hydroxyethyl)-sym-xylidin, N,N,-Diethyl-sym-di-tert-butylanilin), während die andere Paste den spaltbaren Initiator, z.B. ein Diacylperoxid (z.B. Dibenzoylperoxid, Dimethoxydibenzoylperoxid, Dilauroylperoxid, Didodecanoylperoxid, Dilauroylperoxidicarbonat) enthält.

Ebenso ist der Einsatz von lichthärtenden Systemen denkbar. Dieses System besteht aus einer mit Licht polymerisierbaren Masse, welche Fotoinitiatoren, z.B. Phosphinoxide, Triphenylphosphinoxide oder Thioxanthone enthält. Es eignen sich besonders Fotoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie Benzoinether, Dialkylbenzilketale, Dialkoxyacetophenone, Acyl- der Bisacylphosphinoxide, □-diketone wie 9,10-Phenanthrenchinon, Diacetyl, Furil, Anisil, 4,4'-Dichlorbenzil und 4,4'-Dialkoxybenzil und Campherchinon. Weiterhin können auch Azoverbindungen, wie 2,2'-Azobis-(isobutyronitril) (AIBN) oder Azobis-(4-cyanovaleriansäure) eingesetzt werden.

Im Folgenden wird die Erfindung anhand von Beispielen näher beschrieben:

### Beispiel 1: Transparentes Heißpolymerisat für kieferorthodontische Platten oder transparente Gaumenplatten.

### 1. Herstellung einer vorgequollenen Masse

■ 0,65 kg ULTRASON S 3010 (Schmelzflussindex MFI = 8 g/10 min. 343 °C, 2.16 kg, nach ISO 1133) in Granulatform werden in einem Gefäß vorgelegt und mit 0,35 kg Benzylmethacrylat versetzt. Danach wird das Granulat mindestens 20 Std. bei 60 °C in einem Vorwärmofen vorgequollen. Die gequollene Masse wird mit Hilfe eines Z- Arm- Kneters soweit homogenisiert, dass eine nicht klebrige, transparente, knollen- und schlierenfreie Masse vorliegt.

### 2. Herstellung der polymerisierbaren Masse

0,5 kg der vorgequollenen, homogenen Masse werden in einem Z- Arm- Kneter vorgelegt und mit 20 g Benzylmethacrylat verknetet, bis ein homogener Teig entsteht. Um die Masse polymerisationsfähig zu machen, werden 6 g Lauroylperoxid in die vorliegende Masse eingearbeitet.

### 3. Abfüllung

Die Masse wird in einen einseitig verschlossenen Folienschlauch gedrückt. Nach der Füllung wird der Beutel durch Verschweißen oder mit Hilfe von Metallclips verschlossen.

### 4. Anwendung

Eine orthodontische Gaumenplatte aus Wachs wird in eine Metallküvette gemäß der Universal-Presstechnik (Kaneda, K. "Grundkonzept für ästhetische Vollprothese", Quintessenz Zahntechnik 16/7 (1990)) eingebettet. Nach dem Abbinden des Gipses wird die Platte aus Gips mit heißem Wasser ausgebrüht. Nach dem Auskühlen des Gipses wird die Gipsform mit gebräuchlichem Alginattrennmittel isoliert.

Der Folienschlauch wird geöffnet und die benötigte Menge der Masse inklusive eines Überschusses der Verpackung entnommen. Danach wird der Teig in die isolierte Gipshohlform der Küvette gelegt, mit der Konterhälfte versehen und in einer Hydraulikpresse verpresst. Der dabei ausgepresste Überschuss wird verworfen. Die gepresste Küvette wird mit einem Küvettenbügel fixiert und danach in ein heizbares Wasserbad mit kaltem Wasser von 23°C eingestellt. Danach wird das Wasserbad auf 100°C aufgeheizt. Nach Erreichen der Kochtemperatur des Wassers wird die Küvette 45 min. lang gekocht. Danach wird die Küvette dem Bad entnommen und über 30 Minuten bei Raumtemperatur abgekühlt. Nach einer weiteren halben Stunde Abkühlzeit unter fließendem, kaltem Wasser wird die orthodontische Platte ausgebettet.

### Beispiel 2: Rosa eingefärbtes Heißpolymerisat für die Injektionstechnik

### 1. Herstellung von Farbkonzentraten

Dispergierung von Pigmenten mit Hilfe eines pulverförmigen Polysulfones:
■ 14,85 kg UDEL P 1800 NT (MFI = 6-7 g/10 min., 343 °C, 2.16 kg, nach ISO 1133) werden in einen Dierksmischer vorgelegt.
• 0,015 kg eines Eisenoxidpigmentes, eines Azo- oder eines Titandioxidpigmentes werden innerhalb 3 Minuten bei 1000 Umdrehungen/Minute unter Wasserkühlung im Dierksmischer in dem Polysulfon dispergiert.

### 2. Herstellung einer vorgequollenen Masse

0,65 g ULTRASON S 3010 (MFI = 8 g/10 Min., 343 °C, 2.16 kg) in Granulatform werden in einem Gefäß vorgelegt und mit 0,35 kg Benzylmethacrylat versetzt. Danach wird das Granulat mindestens 20 Std. bei 60 °C in einem Vorwärmofen vorgequollen. Die gequollenen Masse wird mit Hilfe eines Z- Arm- Kneters soweit homogenisiert, dass eine nicht klebrige, transparente, knollen- und schlierenfreie Masse vorliegt.

### 3. Herstellung der polymerisierbaren Masse

366 g der vorgequollenen, homogene Masse werden in einem Z- Arm- Kneter vorgelegt und mit 6,6 g Benzylmethacrylat verknetet, bis ein homogener Teig entsteht. Danach werden 3,3 g der Farbkonzentratmischung von 1 zum Kneteransatz gegeben und solange geknetet, bis der Teig eine gleichmäßige Farbe bekommt und schlieren-, und knollenfrei vorliegt.

Um die Masse polymerisationsfähig zu machen werden 0,75 g Lauroylperoxid in die vorliegende Masse eingeknetet, bis eine homogene Paste mit gleichmäßiger, rosa-weißlicher Färbung entsteht.

### 4. Abfüllung

Die Masse wird in einen einseitig verschlossenen Folienschlauch gedrückt. Nach der Füllung wird der Beutel durch Verschweißen oder mit Hilfe von Metallclips verschlossen.

### 5. Anwendung

Vor dem Erstellen der Wachsprothese werden die Zahnhälse der im Gipskonter steckenden Zähne aufgeraut und mit Retentionsvertiefungen versehen. Als zusätzliche Verbesserung der Zahnhaftung werden die Zahnhälse ausgiebig mit reinem Methylmethacrylat bepinselt.

Eine Wachsprothese wird modellseitig in die Küvettenhälfte einer IVOCAP®- Injektionsküvette (DE 23 12 934), eingebettet. Danach wird der Gipskonter erstellt. Sobald der Gips abgebunden ist, wird die Platte aus Wachs mit heißem Wasser ausgebrüht. Nach dem Auskühlen des Gipses werden sämtliche Gipsflächen mit gebräuchlichem Alginattrennmittel isoliert.

Der Folienschlauch wird geöffnet und die benötigte Menge der Masse inklusive einem Überschuss der Verpackung entnommen, in eine leere IVOCAP- Hülse gefüllt und mit dem dazugehörigen Kolben versehen.

Gemäß Kaneda, K. "Grundkonzept für ästhetische Vollprothese", Quintessenz Zahntechnik 16/7 (1990) wird die IVOCAP- Küvette in einen für das IVOCAP- System vorgesehenen Spannrahmen eingespannt und Hülse mit Kolben und Trichter werden danach in den zur Küvette zugehörigen Zentriereinsatz eingeführt.

Der Druckaufsatz wird aufgesetzt und die Masse wird danach in das Küvetteninnere 5 Minuten lang injiziert. Die Küvette wird für 35 Minuten in kochendem Wasser erhitzt. Danach wird die heiße Küvette samt Spannrahmen 30 Minuten lang unter fließendem, kalten Wasser abgekühlt.

Nach dieser Zeit wird die Prothese ausgebettet, ausgearbeitet und poliert.

### Beispiel 3: Selbsthärtendes Zweipastenmaterial

### 1. Herstellung von Farbkonzentraten

- Die Herstellung der Farbkonzentrate erfolgt analog dem vorher beschriebenen Ausführungsbeispiel.

### 2. Herstellung einer vorgequollenen Masse

0,28 kg des ULTRASON S 6010 (MFI = 5 g/10 min., 343 °C, 2.16 kg) oder UDEL P 3500 NT (MFI = 3-5 g/10 min., 343 °C, 2.16 kg) werden in einem Gefäß vorgelegt und mit 0,17 kg Methylmethacrylat versetzt. Danach wird das Granulat mindestens 20 Std. bei 60 °C im Ofen vorgequollen. Nach 20 Std. wird das überstehende Methylmethacrylat abgegossen und verworfen. Die gequollenen Masse wird nun mit Hilfe eines Z- Arm- Kneters solange homogenisiert, bis eine nicht klebrige, transparente, knollen- und schlierenfreie Paste vorliegt.

### 3. Herstellung der Initiator Paste

80 g der vorgequollenen, homogenen Masse werden in einem Z- Arm- Kneter vorgelegt und mit 14 g Methylmethacrylat verdünnt, bis ein homogener Teig entsteht. Danach werden 6,7 g der Farbkonzentrat- Mischung aus 1 zu der Masse gegeben und solange geknetet, bis der Teig eine gleichmäßige Farbe bekommt und schlieren- und knollenfrei vorliegt. Danach werden 16 g SR- 348 C portionenweise in den Teig eingeknetet, bis wiederum eine geschmeidige und homogene Paste resultiert. Um die Masse polymerisationsfähig zu machen, werden 2 g Lauroylperoxid in die vorliegende Masse eingeknetet, bis eine homogene gleichmäßig weißlich gefärbte Paste entsteht.

### 4. Herstellung der Beschleuniger- Paste

80 g der vorgequollenen, homogenen Masse wird in einem Z- Arm- Kneter vorgelegt und mit 14 g Methylmethacrylat verdünnt, bis ein homogener Teig entsteht.

Danach werden 6,7 g der Konzentrat- Mischung aus 1 zu der Masse gegeben und solange geknetet, bis der Teig eine gleichmäßige Farbe bekommt und schließlich schlieren-, und knollenfrei vorliegt. Danach werden 16 g SR- 348 C portionenweise in den Teig eingeknetet, bis wiederum eine geschmeidige und homogene Paste resultiert. Um die Masse polymerisationsfähig zu machen, werden 1 g N-N-Diethanol-p-toluidin in die vorliegende Masse eingeknetet, bis die Kristalle vollständig gelöst sind und eine gleichmäßig gefärbte Paste entsteht.

### 5. Abfüllung

Initiator- oder Beschleuniger- Paste werden separat in einseitig verschlossene Folienschläuche gedrückt. Diese werden schnell gefüllt. Nach der Füllung werden die Beutel wieder verschlossen.

### 6. Anwendung

Vor dem Erstellen der Wachsprothese werden die Zahnhälse der Zähne aufgeraut und mit Retentionsvertiefungen versehen. Als zusätzliche Verbesserung der Zahnhaftung werden die Zahnhälse ausgiebig mit reinem Methylmethacrylat bepinselt.

Eine Wachsprothese wird in die Küvettenhälfte einer Metallküvette eingebettet. Nach dem Abbinden des Gipses wird die Platte aus Gips mit kochend heißem Wasser ausgebrüht. Nach dem Auskühlen des Gipses wird die Gipsform mit gebräuchlichem Alginattrennmittel isoliert.

Die Folienbeutel werden geöffnet und die benötigte Menge jeder Paste zu gleichen Teilen der Verpackungen entnommen. Danach werden die 2 Pasten ca. 60 Sekunden von Hand gemischt. Der gemischte Teig wird in die isolierte Gipshohlform der Küvette gelegt, mit der Konterhälfte versehen und unter der Hydraulikpresse verpresst.

Der dabei ausgepresste Überschuss wird verworfen. Die verpresste Küvette wird in einem Drucktopf unter 2,2 bar mit Wasser von 50°C gestellt und anschließend für 20 Min. polymerisiert. Danach wird die Küvette dem Drucktopf entnommen und 5 Minuten unter fließendem Wasser abgekühlt. Die fertige Prothese wie gewohnt ausgebettet, ausgearbeitet und poliert.

### Beispiel 4: Lichthärtendes Prothesenmaterial

### 1. Herstellung von Farbkonzentraten

Die Herstellung der Farbkonzentrate erfolgt analog dem vorher beschriebenen Ausführungsbeispiel.

### 2. Herstellung einer vorgequollenen Masse

0,25 kg UDEL P 1800 NT werden in einem Gefäß vorgelegt und mit 0,25 kg Phenoxyethylmethacrylat versetzt. Danach wird das Granulat mindestens 20 Std. bei 60 °C in einem Vorwärmofen vorgequollen. Die gequollenen Masse wird mit Hilfe eines Z- Arm- Kneters solange homogenisiert, dass eine nicht klebrige, transparente, knollen- und schlierenfreie Masse vorliegt.

### 3. Herstellung der polymerisierbaren Masse

500 g der vorgequollenen, homogenen Masse werden lichtgeschützt in einem Z-Arm- Kneter vorgelegt und mit 5 g LUCIRIN® TPO versetzt, bis der Photoinitiator gelöst ist und ein homogener Teig entsteht.

### 4. Abfüllung

Der pastöse Teig wird lichtgeschützt unter Vakuum im Kneter entlüftet, und in schwarzen Dosen gelagert. Die Paste kann auch in Folien verpresst werden, um präfabrizierte Platten oder wurstförmige Wälle zu erhalten, welche zur Herstellung von Basisplatten zur Unterfütterung oder als Basis für die Zahnaufstellung verwendet werden.

### 5. Anwendung

Ein Gipsmodell mit basaler Abformung wird erstellt. Dieses wird mit Alginatlösung isoliert. Auf den getrockneten Alginatfilm wird nun eine präfabrizierte Platte gelegt und von Hand an das Gipsmodell adaptiert. Danach wird die geformte Platte für 15 Minuten im SPECTRAMAT® oder im LUMAMAT® (Leistung: Max. 750 Watt, 400- 580 nm, Leuchtstoffröhren, beide IVOCLAR VIVADENT AG, Schaan) samt dem Gipsmodell belichtet. Nach der Belichtung wird die ausgehärtete Platte vom Gipsmodell heruntergenommen und danach mit der zuvor dem Gips zugewandten und nun nach oben der Lichtquelle entgegengerichtet Seite für weitere 15 Minuten belichtet.

Nach der Belichtung können die Zähne direkt auf diese Platte aufgewachst werden. Die Basisplatte dient dann als individueller Löffel, welcher nicht verworfen wird, sondern als Basisplatte für die Prothese dient. Dies ist der Vorteil gegenüber den herkömmlichen Löffelmaterialien, welche für den physiologischen Einsatz nicht verwendbar sind und nach der Einprobe ihre Funktion für das Fertigprodukt verlieren.

### Formulierungen 1: Heißhärtendes Einkomponentenprothesenmaterial

| Komponenten | Gehalt |
|---|---|
| Polysulfon (MFI = 3-18 g/10 min., 343°C, 2.16 kg) UDEL (Solvay^{®} Corporation) oder ULTRASON S, (BASF AG) | 38,5 - 69,35 % |
| Monomethacrylat | 30- 45 % |
| Pigmente | 0,1- 0,3 % |
| Diacylperoxid, Perester | 0,5- 1% |
| Ethoxyliertes Bisphenol A- Dimethacrylat (SR 348 C) | 0- 15 % |
| Tert. Butyl- ortho-Kresol | 0,05-0,2 % |

### Formulierung 2: 2 Komponenten- Pasten-Autopolymerisat

Zusammensetzung der Beschleuniger-Paste

| ***Komponenten*** | ***Gehalt*** |
|---|---|
| Polysulfon ((MFI = 3 - 18 g/10 min., 343°C, 2.16 kg) UDEL (Solvay^{®} Corporation) oder ULTRASON S, (BASF AG) | 37,5 - 69,35 % |
| Monomethacrylat | 30- 45 % |
| Pigmente | 0,1- 0,3 % |
| Tertiäres Amin | 0,5- 2 % |
| SR 348 C | 0- 15 % |
| HALS-Stabilisator | 0,05-0,2 % |

### Zusammensetzung der Initiator-Paste

| ***Komponenten*** | ***Gehalt*** |
|---|---|
| Polysulfon (MFI = 3 - 18 g/10 min., 343°C, 2.16 kg) UDEL (Solvay^{®} Corporation) oder ULTRASON S, (BASF AG) | 37,8 - 69,45 % |
| Monomethacrylat | 30- 45 % |
| Pigmente | 0,1- 0,3 % |
| Diacylperoxid | 0,5- 2 % |
| SR348C | 0-15% |
| Tert. Butyl- ortho-Kresol | 0,05-0,2 % |

### Formulierungen 3: Lichthärtendes Einkomponentenprothesenmaterial

| ***Komponenten*** | ***Gehalt*** |
|---|---|
| Polysulfon (MFI = 3 - 18 g/10 min., 343°C, 2.16 kg) UDEL (Solvay^{®} Corporation) oder ULTRASON S, (BASF AG) | 37,8 - 69,45 % |
| Monomethacrylat | 30- 45 % |
| Pigmente | 0,1- 0,3 % |
| Triphenylphosphinoxid oder Campherquinone | 0,5- 2 % |
| SR 348 C | 0-15% |

### Physikalische Eigenschaften

- Biegefestigkeit und Biegemodul nach ISO 1567:1999 Beispiele 2- 4

| ***Varianten*** | **Biegefestigkeit in *[MPa]*** | **E- Modul in *[MPa]*** |
|---|---|---|
| *Beispiel 2:* Eingefärbtes heißhärtendes Einkomponentenmaterial, z.B. für die Injektionstechnik | 98 ± 1 | 3055 ± 40 |
| *Beispiel 3:* Selbsthärtendes Zweipastenmaterial | 85 ± 5 | 2710 ± 95 |
| *Beispiel 4:* Lichthärtendes Prothesenmaterial | 86 ± 0,5 | 2750 ± 55 |

- Fracture Toughness nach ISO 20795- Part 1 (Working Draft 2005)

| ***Varianten*** | **Fracture Work in *J*/*m²*** | ***Kₘₐₓ-Wert in [MPa*m*^{*1*/}*²]*** |
|---|---|---|
| *Beispiel 2:* Eingefärbtes Heißhärtendes Einkomponentenmaterial z.B. für die Injektionstechnik | 840 ± 225 | 2,2 ± 0,1 |
| *Beispiel 3:* Selbsthärtendes Zweipastenmaterial | 1225 ± 195 | 2,9 ± 0,1 |
| *Beispiel 4:* Lichthärtendes Prothesenmaterial | 250 ± 45 | 1,6 ± 0,1 |

■ Vergleich mit dem Stand der Technik:
-- Biegefestigkeit und Biegemodul nach ISO 20795-1

| ***Variante*** | **Biegefestigkeit in *[MPa]*** | **E- Modul in *[MPa]*** |
|---|---|---|
| Heißhärtendes Einkomponentenmaterial PURAN^{®} HC, (NOVODENT Schaan) | 63.,5 ± 6,5 | 2000 ± 50 |
| Heißhärtendes Einkomponentenmaterial für die Injektionstechnik Injectall HI-I/C-F, (FRICKE Corp.) | 78 ±1,5 | 2280 ± 45 |
| Autopolymerisat auf PMMA-Basis, ProBase cold, (IVOCLAR VIVADENT AG) | 68 | 2550 |
| Lichthärtendes Prothesenmaterial VERSYO.COM (Heraeus Kulzer GmbH) | 108,5 ± 5,5 | 2810 ± 75 |

-- Fracture Toughness nach ISO 20795- Part 1

| ***Varianten*** | **Fracture Work in J/m**² | ***Kₘₐₓ- Wert in IMPa*m* ^{*1*/}*²]*** |
|---|---|---|
| Heißhärtendes Einkomponentenmaterial PURAN^{®} HC, (NOVODENT Schaan) | 82 ± 3 | 0,68 ± 0,04 |
| Heißhärtendes Einkomponentenmaterial Für Injektionstechnik Injectall HI-I/C-F, (FRICKE Corp.) | 940 ± 90 | 1,95 ± 0,1 |
| Autopolymerisat auf PMMA- Basis, ProBase cold, (IVOCLAR VIVADENT) | 400 ± 100 | 1,5 ± 0,1 |
| Lichthärtendes Prothesenmaterial (VERSYO.com) (Heraeus Kulzer GmbH) | 60 ±10 | 0,77 ± 0,14 |

## Patentansprüche

1. Verformbare Massen enthaltend eine Mischung aus
20 bis 50 Gew.-% wenigstens eines polymerisationsfähigen, für Dentalzwecke geeigneten Monomers und
50 bis 80 Gew.-% wenigstens eines für Dentalzwecke geeigneten Polymers auf Basis von Polysulfon, das wenigstens teilweise in wenigstens einem Teil des Monomers gequollen ist.
wobei sich die Anteile auf jeweils 100 Gew.-% addieren und wobei die Masse als polymerisationsfähige Monomere Mono(meth)acrylate oder kurzkettige Di(meth)acrylate oder Gemische hiervon enthält..

2. Verformbare Masse nach Anspruch 1, **dadurch gekennzeichnet, dass** sie 25 bis 45 Gew.-% Monomer und 55 bis 75 Gew.-% Polymer enthalten, wobei sich die Anteile auf jeweils 100 Gew.-% addieren.

3. Verformbare Masse nach einem der vorhergehenden Ansprüche, **dadurch gekennzeichnet, dass** sie als polymerisationsfähige Monomere (Meth)Acrylate enthält.

4. Verformbare Masse nach einem der vorhergehenden Ansprüche, **dadurch gekennzeichnet, dass** sie Initiatoren enthält.

5. Verformbare Masse nach einem der vorhergehenden Ansprüche, **dadurch gekennzeichnet, dass** sie durch Energiezufuhr aktivierbare Initiatoren enthält.

6. Verformbare Masse nach Anspruch 5, **dadurch gekennzeichnet, dass** sie als Initiatoren Peroxide enthält.

7. Verformbare Masse nach einem der vorhergehenden Ansprüche, **dadurch gekennzeichnet, dass** sie Aktivatoren enthält.

8. Verformbare Masse nach Anspruch 7, **dadurch gekennzeichnet, dass** sie als Aktivatoren Amine oder Gemische daraus enthält.

9. Verformbare Masse nach einem der Ansprüche 1 bis 8, **dadurch gekennzeichnet, dass** sie Fotoinitiatoren enthält.

10. Verformbare Masse nach Anspruch 9, **dadurch gekennzeichnet, dass** sie als Fotoinitiatoren Campherquinon, Azoverbindungen, Phosphinoxide oder Thioxanthone enthält.

11. Verformbare Masse nach einem der vorhergehenden Ansprüche, **dadurch gekennzeichnet, dass** die Initiatoren und/oder Aktivatoren stabilisiert oder phlegmatisiert sind.

12. Verfahren zur Herstellung von verformbaren Massen nach einem der Ansprüche 1 bis 11, **dadurch gekennzeichnet, dass**
- 20 bis 50 Gew.-% wenigstens eines polymerisationsfähigen, für Dentalzwecke geeigneten Monomers,
- 50 bis 80 Gew.-% wenigstens eines für Dentalzwecke geeigneten Polymers auf Basis von Polysulfon derart miteinander vermischt werden, und
- wenigstens teilweise das Polysulfon in wenigstens einem Teil des Monomers gequollen wird.
wobei sich die Anteile auf jeweils 100 Gew.-% addieren.

13. Verfahren nach Anspruch 12, **dadurch gekennzeichnet, dass**
25 bis 45 Gew.-% Monomer und
55 bis 75 Gew.-% Polymer eingesetzt werden,
wobei sich die Anteile auf jeweils 100 Gew.-% addieren.

14. Verwendung der verformbaren Massen nach einem der Ansprüche 1 bis 11 zur Herstellung von Dentalprodukten, vorzugsweise Dentalprothesen.

## Claims

1. Moldable materials comprising a mixture of:
from 20 to 50% by weight of at least one polymerizable monomer suitable for dental purposes and from 50 to 80% by weight of at least one polysulfone-based polymer suitable for dental purposes which is at least partially swollen in at least a portion of the monomer,
the amounts adding up to 100% by weight each time and the material comprising, as polymerizable monomers, mono(meth)acrylates or short-chain di(meth)acrylates or mixtures thereof.

2. Moldable material according to Claim 1, **characterized in that** it comprises from 25 to 45% by weight of monomer and from 55 to 75% by weight of polymer, the amounts adding up to 100% by weight each time.

3. Moldable material according to either of the preceding claims, **characterized in that** it comprises (meth)acrylates as polymerizable monomers.

4. Moldable material according to one of the preceding claims, **characterized in that** it comprises initiators.

5. Moldable material according to one of the preceding claims, **characterized in that** it comprises initiators which can be activated by introduction of energy.

6. Moldable material according to Claim 5, **characterized in that** it comprises peroxides as initiators.

7. Moldable material according to one of the preceding claims, **characterized in that** it comprises activators.

8. Moldable material according to Claim 7, **characterized in that** it comprises, as activators, amines or mixtures thereof.

9. Moldable material according to one of Claims 1 to 8, **characterized in that** it comprises photoinitiators.

10. Moldable material according to Claim 9, **characterized in that** it comprises, as photoinitiators, camphorquinone, azo compounds, phosphine oxides or thioxanthones.

11. Moldable material according to one of the preceding claims, **characterized in that** the initiators and/or activators are stabilized or inhibited.

12. Process for the preparation of moldable materials according to one of Claims 1 to 11, **characterized in that**
- from 20 to 50% by weight of at least one polymerizable monomer suitable for dental purposes and
- from 50 to 80% by weight of at least one polysulfone-based polymer suitable for dental purposes are mixed with one another, and
- the polysulfone is at least partially swollen in at least a portion of the monomer,
the amounts adding up to 100% by weight each time.

13. Process according to Claim 12, **characterized in that**
from 25 to 45% by weight of monomer and
from 55 to 75% by weight of polymer are used,
the amounts adding up to 100% by weight each time.

14. Use of the moldable materials according to one of Claims 1 to 11 in the preparation of dental products, preferably dental prostheses.

## Revendications

1. Masses déformables, contenant un mélange de
- 20 à 50% en poids d'au moins un monomère polymérisable, approprié à des fins dentaires et
- 50 à 80% en poids d'au moins un polymère approprié à des fins dentaires à base de polysulfone, qui est gonflé au moins partiellement dans au moins une partie du monomère,
où les proportions s'ajoutent à chaque fois à 100% en poids et où la masse contient, comme monomères polymérisables, des mono(méth)acrylates ou des di(méth)acrylates à courte chaîne ou des mélanges de ceux-ci.

2. Masse déformable selon la revendication 1, **caractérisée en ce qu'**elle contient 25 à 45% en poids de monomère et 55 à 75% en poids de polymère, où les proportions s'ajoutent à chaque fois à 100% en poids.

3. Masse déformable selon l'une quelconque des revendications précédentes, **caractérisée en ce qu'**elle contient des (méth)acrylates comme monomères polymérisables.

4. Masse déformable selon l'une quelconque des revendications précédentes, **caractérisée en ce qu'**elle contient des initiateurs.

5. Masse déformable selon l'une quelconque des revendications précédentes, **caractérisée en ce qu'**elle contient des initiateurs activables par apport d'énergie.

6. Masse déformable selon la revendication 5, **caractérisée en ce qu'**elle contient des peroxydes comme initiateurs.

7. Masse déformable selon l'une quelconque des revendications précédentes, **caractérisée en ce qu'**elle contient des activateurs.

8. Masse déformable selon la revendication 7, **caractérisée en ce qu'**elle contient des amines ou des mélanges d'amines comme activateurs.

9. Masse déformable selon l'une quelconque des revendications 1 à 8, **caractérisée en ce qu'**elle contient des photo-initiateurs.

10. Masse déformable selon la revendication 9, **caractérisée en ce qu'**elle contient de la camphrequinone, des composés azo, des oxydes de phosphine ou des thioxanthones comme photo-initiateurs.

11. Masse déformable selon l'une quelconque des revendications précédentes, **caractérisée en ce que** les initiateurs et/ou les activateurs sont stabilisés ou flegmatisés.

12. Procédé pour la préparation de masses déformables selon l'une quelconque des revendications 1 à 11, **caractérisé en ce que**
- 20 à 50% en poids d'au moins un monomère polymérisable, approprié à des fins dentaires
- 50 à 80% en poids d'au moins un polymère approprié à des fins dentaires à base de polysulfone sont mélangés les uns avec les autres et
- la polysulfone est gonflée au moins partiellement dans au moins une partie du monomère,
où les proportions s'ajoutent à chaque fois à 100% en poids.

13. Procédé selon la revendication 12, **caractérisé en ce que**
- 25 à 45% en poids de monomère et
- 55 à 75% en poids de polymère sont utilisés,
où les proportions s'ajoutent à chaque fois à 100% en poids.

14. Utilisation des masses déformables selon l'une quelconque des revendications 1 à 11 pour la fabrication de produits dentaires, de préférence des prothèses dentaires.
